# EUROPEAN PATENT APPLICATION

(11) **EP 1 016 410 A1**
(43) Date of publication of application: **05.07.2000**
(21) Application number: 99650121.9
(22) Date of filing: 23.12.1999
(51) Int. Cl.: A61K 31/726, A61K 31/727, A61P 43/00

(54) **Glycosaminoglycans for the treatment for pre-eclampsia and related diseases**

(30) Priority: 31.12.1998 IL 12789698
(71) Applicant: Lakaro Biopharmaceutical, Inc., Jerusalem 94383 (IL)
(72) Inventor: Laster, Gail, Jesusalem 97289 (IL)
(74) Representative: McCarthy, Denis Alexis

(57) **Abstract**

A composition for treating a disease associated with underlying endothelial damage in a pregnant subject. The composition includes a pharmaceutically effective amount of a glycosaminoglycan, preferably sulodexide.

## Description

### FIELD AND BACKGROUND OF THE INVENTION

The present invention relates to a novel treatment for diseases in pregnancy associated with underlying endothelial damage, such as pre-eclampsia or intrauterine growth restriction (IUGR). In particular, it concerns the effect of a glycosaminoglycan, preferably a mixture of heparan or heparin sulfate and dermatan sulfate, such as Sulodexide for the treatment of pre-eclampsia, complications of pre-eclampsia and as a prophylactic treatment of impending or suspected pre-eclampsia.

Sulodexide is a glycosaminoglycan with unique reparative effects in endothelial structure and function. Other examples of glycosaminoglycans include heparin or heparan and its salts, low molecular weight heparins or heparans, chemically modified heparins or heparans and dermatan sulfate. Sulodexide is a biological extract compound comprised of approximately 80% heparan or heparin sulfate and 20% dermatan sulfate. Native heparan or heparin sulfate serves several functions in endothelial membranes, including anti-proliferative functions, e.g., it suppresses arterial wall smooth muscle cell proliferation, and anti-thrombitic functions, e.g., it activates anti-thrombin III. Dermatan sulfate activates heparan cofactor 2.

Bulvas, *et al.* (1) showed that heparin sulfate administration to patients following PTCA due to stenosis of the aortoiliac or femoropopliteal area improved several angiological parameters.

Indeed, a large double-blind, multicenter Italian study published in 1990 by Crepalodi, *et al.* (2) revealed a statistically significant improvement in the long term treatment of peripheral vascular disease and a concomitant increase in plasma triglycerides, fibrinogen and/or viscosity.

Condorelli, *et al.* (3) published a prospective, multicenter, randomised trial assessing the effects of Sulodexide in preventing cardiovascular events following myocardial infarction. These researchers found that long-term therapy with Sulodexide started early after an episode of acute myocardial infarction is associated with reductions in total mortality, rate of reinfarcation and mural thrombus formation.

Recently, Sulodexide was assessed in the treatment of diabetic nephropathy (4-6). Several studies in over 150 patients have shown that Sulodexide diminishes urinary albumin excretion which is believed to result from an endothelial defect. The beneficial effect is achieved by supplementing vascular wall heparan sulfate and suppressing mesangial cell proliferation. Finally, Park, *et al.* (7) showed that Sulodexide was effective in the inhibition of neointimal proliferation after vascular injury in an *in vivo* rat model.

However, none of these prior art references suggested the use of glycosaminoglycans for the treatment of pre-eclampsia, its complications or related diseases.

Pre-eclampsia (PE) is a multisystem disorder of human pregnancy. It is a progressive disease which afflicts approximately 7% of all pregnancies. Its manifestations are considered secondary to organ hypoperfusion, which arises as a result of vasoconstriction, intravascular coagulation and reduced maternal blood volume. PE is caused by widespread maternal endothelial cell damage, which is probably secondary to abnormal trophoblast invasion of the uterine parenehyma during implantation (8).

Endothelial cells are strategically located to serve as sensory cells assessing hemodynamic and humoral signals, as well as effector cells eliciting biological responses that may eventually affect the structure of the underlying vessel wall. To maintain the patency of blood vessels and the fluidity of blood, endothelial cells synthesize many active substances, including large molecules such as fibronectin, heparan sulfate, tissue plasminogen activator (tPA) and smaller molecules such as prostacyclin (PGI₂), endothelium-derived relaxing factor (EDRF) and endothelin. Endothelial cells modulate the reactivity of underlying vascular smooth muscle in response to vasoactive stimuli (9).

Endothelial cell dysfunction plays an important role in the pathogenesis of PE. Increased levels of fibronectin, an abnormal tPA balance and a disturbance of the PGI₂/Thromboxane balance all support the hypotheses that endothelial cell dysfunction is intimately involved in the pathogenesis of PE. Morphological evidence of endothelial cell injury is provided by the characteristic lesions of PE: glomerular endotheliosis and ultrastructural changes in placental bed and uterine boundary vessels (10).

Endothelial cell dysfunction is the final common pathway in the pathogenesis of PE. The exact cause of this dysfunction is uncertain, however several mechanisms have been suggested:
1. Abnormal trophoblast invasion of maternal spiral arteries with ensuing placental ischemia. This results in release of a cytotoxic placental factor which suppresses endothelial cell proliferation.
2. Excess very low density lipoprotein (VLDL) concentrations to offset energy demands not met by increased mobilization of non-esterified fatty acids. VLDL toxicity causes injury to endothelial cells.
3. Excessive neutrophil activation with the resulting release of elastase and other toxic proteases, can destroy the integrity of the endothelium, vascular basement membrane and subendothelial matrix.
4. Placental lipid peroxides may increase the local production of thromboxane and decrease PGI₂, thereby causing progressive vasoconstriction in the placental vasculature.
5. Ongoing lipid-peroxidation of circulating lipids eventually results in disseminated endothelial damage.
6. Endothelial generation of superoxide anions appears to be a key event resulting in endothelial cytotoxicity following exposure to activated neutrophils.

Endothelial cell abnormalities in pre-eclampsia include swollen glomerular capillary endothelial cells, an increase in the level of circulating endothelial derived fibronectin and vWF, increased Evans Blue dye disappearance rate, increased endothelial cell cytotoxicity by serum of PE women, increased lipid peroxidation, local thrombosis, increased platelet-bound fibrinogen, increased plasma and placental PAI-1 and reduced prostacyclin. While normal pregnancy is characterized by increased in vivo thrombin generation, a greater proportion of endogenously generated thrombin is found in PE. Recently, a significant role for placental dermatan sulfate was discovered in the local regulation of thrombin in the placenta (11).

Medication, such as magnesium sulfate and low dose aspirin have been used in an attempt to treat pre-eclampsia and its complications. The complications include endothelial and glomerular basement membrane damage, thrombosis, increased fibrinogen, decreased platelets, elevated liver function tests, increased hematocrat, increased serum creatinine and decreased urine output. However, these drugs are not effective in treating the pre-eclampsia. Aspirin is no longer prescribed for pre-eclampsia and magnesium sulfate is only used in the prevention of seizures. The only accepted therapy for pre-eclampsia is delivery of the placenta, necessitating delivery of the fetus regardless of gestational age or estimated fetal weight. Other attempts to reverse the adverse consequences of endothelial cell dysfunction in pre-eclampsia have been unsuccessful.

Without wishing to be limited by a single hypothesis, presumably the mechanism of pre-eclampsia involves abnormal trophoblast implantation resulting in placental ischemia with concomitant vasoconstriction and widespread endothelial dysfunction. The widespread endothelial damage and impaired glomerular basement membrane function results in hypertension, protein leakage and peripheral edema. The underlying endothelial damage may ultimately result in widespread organ dysfunction including hepatic necrosis, acute tubular necrosis, pulmonary edema and seizures. In addition, the placental ischemia results in localized thrombin generation and decreased utero-placental blood flow with the resulting deleterious consequences for the fetus.

There is thus a widely recognized need for, and it would be highly advantageous to have, a drug which is effective in the possible prevention and treatment of pre-eclampsia.

### SUMMARY OF THE INVENTION

The present invention provides a composition of a glycosaminoglycan, preferably a mixture of heparan or heparin sulfate and dermatan sulfate, and most preferably Sulodexide for the treatment of diseases in pregnancy associated with suspected underlying endothelial damage, such as pre-eclampsia.

According to the teachings of the present invention there is provided in a first embodiment a composition for treating a disease associated with underlying endothelial damage in a pregnant subject, the composition comprising a pharmaceutically effective amount of a glycosaminoglycan.

In a preferred embodiment the disease is pre-eclampsia.

In a preferred embodiment the glycosaminoglycan is selected from the group of low molecular weight heparin or heparan derivatives, chemically modified heparin or heparan derivatives and low molecular weight dermatan sulfates and mixtures thereof.

In a preferred embodiment the glycosaminoglycan is a mixture of heparan or heparin sulfate and dermatan sulfate in a ratio of from about 1 to about 10 to about 10 to about 1 heparan or heparin sulfate to dermatan sulfate.

In a more preferred embodiment the glycosaminoglycan is a mixture of heparan or heparin sulfate and dermatan sulfate in a ratio of from about 4 to about 1 heparan or heparin sulfate to dermatan sulfate.

In a preferred embodiment the glycosaminoglycan is Sulodexide.

In a preferred embodiment the composition is in a tablet form.

In a preferred embodiment the composition is in an intravenous form.

In a preferred embodiment the preferred routes of administration are oral or subcutaneous or parenteral.

In a preferred embodiment the subject is a human.

In a preferred embodiment the subject is a mammal.

In a preferred embodiment the subject is a lower mammal.

In a second embodiment the present invention provides a composition comprising a pharmaceutically effective amount of a glycosaminoglycan to treat a complication of pre-eclampsia in a subject.

In a preferred embodiment the complication is selected from the group consisting of endothelial and glomerular basement membrane damage, thrombosis, increased fibrinogen, decreased platelets, elevated liver function tests, increased hematocrat, increased serum creatinine, proteinurea and decreased urine output.

In a preferred embodiment the glycosaminoglycan for treating a complication of pre-eclampsia is selected from the group of low molecular weight heparin or heparan derivatives, chemically modified heparin or heparan derivatives and low molecular weight dermatan sulfates and mixtures thereof.

In a preferred embodiment the glycosaminoglycan for treating a complication of pre-eclampsia includes Sulodexide.

In a preferred embodiment the composition for treating a complication of pre-eclampsia is in a tablet form.

In a preferred embodiment the composition for treating a complication of pre-eclampsia is in a form for subcutaneous administration.

In a preferred embodiment the composition for treating a complication of pre-eclampsia is in a form for intravenous administration.

In a third embodiment the present invention provides a composition for prophylactic treatment of a disease associated with underlying endothelial damage in a pregnant subject, the composition comprising a pharmaceutically effective amount of a glycosaminoglycan.

In a fourth embodiment the present invention provides a composition for the treatment of fetal growth restriction associated with underlying endothelial dysfunction.

The term 'treating' as used herein refers to both preventative treatment and treatment of the disease.

The term 'disease associated with underlying endothelial damage' as used herein refers to pre-eclampsia and related disorders.

The term 'pre-eclampsia' as used herein refers to pre-eclampsia and complications resulting from pre-eclampsia.

The term 'subject' as used herein refers to humans, mammals and lower mammals.

### DESCRIPTION OF THE PREFERRED EMBODIMENTS

The present invention is of a composition of a glycosaminoglycan, preferably a low molecular weight heparin or heparan derivative, or a chemically modified heparin or heparan derivative, or a low molecular weight dermatan sulfate, or mixtures thereof, more preferably a mixture of heparan or heparin sulfate and dermatan sulfate, and most preferably Sulodexide for the treatment of pre-eclampsia and related diseases in pregnancy associated with underlying endothelial damage. No effective treatment of pre-eclampsia, a condition affecting 7% of all pregnancies, is available. Consequently, there is a tremendous need for a treatment such as is provided in the present invention for use in pre-eclampsia.

It is to be understood that the invention is not limited in its application to the details of construction and the arrangement of the components set forth in the following description. The invention includes other embodiments and can be practiced or implemented in various ways. Also it is to be understood that the phraseology and terminology employed herein is for the purpose of description only and should not be regarded as limiting.

Sulodexide has been shown to have anticoagulant and antithrombotic actions in experimental animals, has oral availability and is used in the treatment of vascular disease. Sulodexide essentially catalyses the inhibition of thrombin generation in situ similar to low molecular weight heparin. Sulodexide has the advantage over heparin of causing much less bleeding, which is undesirable in a pregnant subject. The persistent binding of Sulodexide to the endothelium may account for its mild anti-coagulant pharmacodynamic profile. Its anti-thrombotic activity arises from the apparent ability of this drug to enhance endogenous fibrinolysis in vivo. This is consistent with its ability to decrease the plasma concentration of PAI-1 (12).

Endothelial cell abnormalities in pre-eclampsia include increased circulating endothelial derived fibronectin and vWF, local thrombosis, decreased anti-thrombin III, increased platelet-bound fibrinogen, increased plasma and placental PAI-1, reduced tissue plasminogen activator, increased VLDL levels, increased glomerular basement membrane thickening, abnormal mesangial cell proliferation and damaged ionic charge barrier allowing protein leakage. Sulodexide has been shown to reverse all these endothelial cell abnormalities. Additionally, dermatan sulfate, a primary component of Sulodexide, is involved in the local regulation of thrombin in the placenta.

Sulodexide's clinically tested ability to repair the underlying endothelial and glomerular basement membranes, while preventing thrombosis and enhancing fibrinolysis may therefore result in an amelioration of pre-eclampsia.

It is also anticipated that Sulodexide will have a positive effect on fetal growth restriction caused by underlying endothelial dysfunction or thrombosis.

According to the present invention, an effective amount of a glycosaminoglycan such as Sulodexide can be administered to pregnant subjects exhibiting established pre-eclampsia symptoms, such as elevated blood pressure and increased urinary albumin excretion, or patients at risk. All formulations of Sulodexide are possible, however in each case the glycosaminoglycan, such as Sulodexide is preferably the only active ingredient. The Sulodexide can be administered intravenously, orally, intramuscularly, parenterally, subcutaneously, nasally or rectally. The more preferred routes of administration are oral, subcutaneous or parenteral and the most preferred route is oral.

The present invention may be better understood with reference to the examples and the accompanying description. In the examples any glycosaminoglycan can be used to treat the pre-eclampsia, however for descriptive purposes only Sulodexide is used. This is not intended to be in any way limiting.

### Example 1

An animal model of human pre-eclampsia is created using an ultra low-dose endotoxin infusion in pregnant rats (13). Two groups of rats receive a permanent jugular vein cannula on day 0 of pregnancy. The control group receive an infusion of saline solution (3 ml) over 1 hour on day 14 of pregnancy. The experimental group receive 1.0 micrograms/kg body weight of endotoxin over 1 hour on day 14. This experimental group is further divided into 2 groups on day 15 after establishment of pre-eclampsia, which is detected by (a) an increase of blood pressure and (b) increased urinary albumin excretion. One group receives a saline solution (3 ml for 3 days and then 1 ml for 7 days) via the jugular vein cannula and the second group receives an equivalent volume of Sulodexide (10 mg/kg/day for 3 days and then 4 mg/kg/day for 7 days). Daily blood pressure, urinary albumin excretion, platelet counts and liver function tests are measured. Half the mice in each group are sacrificed following 10 days of treatment and complete histopathological studies are performed. Particular emphasis is placed on examination of the animal for glomerular endotheliosis, periportal hemorrhagic necrosis in the periphery of the liver lobule, cerebral edema and hyperemia and histological changes in the placental bed including acute atherosis. The remaining mice are followed until delivery and complete histopathological examination is performed at that time.

Examinations are conducted for evidence of maternal and fetal reduction of the consequences of pre-eclampsia. These examinations include tests to measure reduced blood pressure, reduced urinary albumin excretion, normal platelet counts, normal liver function tests and normal fibrinogen levels. Additional tests measure normal antithrombin III and vWF and normal gravid prostacyclin levels. Examinations are conducted for testing the absence of characteristic pathologic lesions, such as glomerular endotheliosis and an absence of fetal morbidity, such as intrauterine growth restriction.

### Example 2

An animal model is used to create a superimposed pre-eclampsia like syndrome by reducing uteroplacental blood flow in spontaneously hypertensive rats using a silver clip (14). Urinary excretion of 2,3-dinor-6-keto-PGF1 alpha is measured by enzyme immunoassays at day 16 of pregnancy as a baseline. The rats with a model pre-eclampsia show a reduction in systemic prostacyclin synthesis to non gravid levels. These pre-eclamptic mice are then divided into a treatment group and a control group, receiving Sulodexide and saline, respectively. A control group (gravid, sham-operated animals) is likewise divided into two groups. Levels of prostacyclin, antithrombin III, fibronectin and vWF are measured on days 16, 20 and 24. Complete histopathological examinations are conducted on day 25. Histopathological examination of the placenta is used to confirm the diagnosis of pre-eclampsia.

Examinations are conducted for evidence of maternal and fetal reduction of the consequences of pre-eclampsia. These examinations include tests to measure reduced blood pressure, reduced urinary albumin excretion, normal platelet counts, normal liver function tests and normal fibrinogen levels. Additional tests measure normal antithrombin 111 and vWF and normal gravid prostacyclin levels. Examinations are conducted for testing the absence of characteristic pathologic lesions, such as glomerular endotheliosis and an absence of fetal morbidity, such as intrauterine growth restriction.

### Example 3

### Possible methods of treatment and compositions for administration for the treatment of pre-eclampsia

Sulodexide can be administered to a subject in a number of ways, which are well known in the art. For example administration may be done orally, intravenously, subcutaneously, intramuscularly, parenterally, nasally or rectally. The more preferred routes of administration are oral or subcutaneous and the most preferred route is oral.

Sulodexide can be administered to subjects with diagnosed pre-eclampsia. Additionally, it can be given to subjects at risk, or to those with a symptom indicative of pre-eclampsia, such as elevated blood pressure or increased protein concentration in the urine.

### Various compositions for the treatment of pre-eclampsia as well as satellite syndromes or complications

Compositions for oral administration, which is a preferred route of administration, can be in a form that include powders or granules, suspensions or solutions in water or non-aqueous media, sachets, capsules, tablets, gelcaps and sustained release formulations. Thickeners, diluents, flavorings, vitamins dispersing aids, emulsifiers or binders may be desirable.

All kinds of pharmaceutical compositions administerable by subcutaneous routes can be advantageously used in the present invention.

Compositions for intravenous administration, can be in a form that includes liquid suspensions or solutions in water or non-aqueous media.

Dosing is dependent on the responsiveness of the subject to Sulodexide. The amount received by the subject is controlled. For example as a pill, the dose and frequency of dosing would be dependent on the responsiveness of the subject. Persons of ordinary skill in the art can easily determine optimum dosages, dosing methodologies and repetition rates.

It will be appreciated that the above examples and descriptions are intended only to serve as examples, and that many other embodiments are possible within the spirit and the scope of the present invention.

### REFERENCES CITED

1. Bulvas M, Chochola M, Herdova J et al: The effect of heparin sulfate on percutaneous transluminal angioplasty in the vessels supplying the lower extremities. Cas Kek Cesk 135 (14):445-9, 1996.
2. Crepaldi G, Fellin R, Calabro A et al: Double-blind multicenter trial on a new medium molecular weight glycosaminoglycan. Atherosclerosis 81:233-43, 1990.
3. Condorelli M, Chiariello M, Digianti A, et al: IPO-V2: A prospective, multicenter, randomized, comparative clinical investigation of the effects of Sulodexide in preventing cardiovascular accidents in the first year after acute myocardial infarction. J Am Coll Cardiol 23 (1); 27-34, 1994.
4. Poplawski A, Sxclachowska M, Topolska J, et al: Effect of glycosaminoglycans on urinary albumin excretion in insulin-dependent diabetic patients with micro-or macoalbuminuria. Diabetes Res Clin Pract 38(2):109-14, 1997.
5. Solini A, Vergnani L, Ricci F, et al: Glycosaminoglycans delay the progression of nephropathy in NIDDM. Diabetes Care 20: 819-23, 1997.
6. Dedov I, Shestakova, Vorontzov A, et al: A randomized controlled study of Sulodexide therapy fort the treatment of diabetic nephropathy. Nephrol Dial Transplant 12: 1-6, 1997.
7. Park HY, Kang S, Kim Gy et al: Inhibition of neointimal proliferation of rat carotid artery by Sulodexide. J Korean Med Sci 12 (3): 210-14, 1997.
8. Brown M: The physiology of pre-eclampsia. Clinical Exp Pharmacol Physiol 22:781-91, 1995.
9. Roberts J, Taylor R, Goldfien A: Clinical and biochemical evidence of endothelial cell dysfunction in the pregnancy syndrome pre-eclampsia. AJH 4:700-08, 1991.
10. Ghidini A, Salafia CM, Pezzullo JC: Placental vascular lesions and likelihood of pre-eclampsia. Obstet Gynecol 90:542-45, 1997.
11. Delorme MA, Xu L, Berry L, Mitchell L, Andrew M: Anticoagulant dermatan sulfate proteoglycan in the term human placenta. Thromb Res 90: 147-53, 1998.
12. Ofosu F: Pharmacological actions of Sulodexide. Sem Thromb Hemostas 24 (2):127-38, 1998.
13. Faas MM, Schuiling GA, Baller JF, Visscher CA, Bakker WW: A new animal model for human pre-eclampsia: ultra low-dose endotoxin infusion in pregnant rats. Am J Obstet Gynecol 171:158-64, 1994.
14. Schaefer WR, Seufert RJ, Casper FW, Zahradnik HP: Urinary excretion of 2,3-dinor-6-keto-PGF1 alpha and 11-dehydro-TXB2 by the gravid spontaneously hypertensive rate. Prostaglandins 52 (1):1-11, 1996.

## Claims

1. A composition for treating a disease associated with underlying endothelial damage in a pregnant subject, the composition comprising a pharmaceutically effective amount of a glycosaminoglycan.

2. The composition of claim 1, wherein said disease is pre-eclampsia.

3. The composition of claim 1, wherein said glycosaminoglycan is selected from the group of low molecular weight heparin or heparan derivatives, chemically modified heparin or heparan derivatives and low molecular weight dermatan sulfates and mixtures thereof.

4. The composition of claim 1, wherein said glycosaminoglycan is a mixture of heparan or heparin sulfate and dermatan sulfate in a ratio of from about 1 to about 10 to about 10 to about 1 heparan or heparin sulfate to dermatan sulfate.

5. The composition of claim 1, wherein said glycosaminoglycan is most preferably a mixture of heparan or heparin sulfate and dermatan sulfate in a ratio of from about 4 to about 1 heparan or heparin sulfate to dermatan sulfate.

6. The composition of claim 1, wherein said glycosaminoglycan is Sulodexide.

7. The composition of claim 1, provided in a tablet form.

8. The composition of claim 1, provided in an intravenous form.

9. The composition of claim 1, wherein preferred routes of administration are oral or subcutaneous or parenteral.

10. The composition of claim 1, wherein said subject is a human.

11. The composition of claim 1, wherein said subject is a mammal.

12. The composition of claim 11, wherein said subject is a lower mammal.

13. A composition comprising a pharmaceutically effective amount of a glycosaminoglycan to treat a complication of pre-eclampsia in a subject.

14. The composition of claim 13, wherein said complication is selected from the group consisting of endothelial and glomerular basement membrane damage, thrombosis, increased fibrinogen, decreased platelets, elevated liver function tests, increased hematocrat, increased serum creatinine, proteinurea and decreased urine output.

15. The composition of claim 13, wherein said glycosaminoglycan is selected from the group of low molecular weight heparin or heparan derivatives, chemically modified heparin or heparan derivatives and low molecular weight dermatan sulfates and mixtures thereof.

16. The composition of claim 13, wherein said glycosaminoglycan includes Sulodexide.

17. The composition of claim 13, provided in a tablet form.

18. The composition of claim 13, provided in a form for subcutaneous administration.

19. The composition of claim 13, provided in a form for intravenous administration.

20. A composition for prophylactic treatment of a disease associated with underlying endothelial damage in a pregnant subject, the composition comprising a pharmaceutically effective amount of a glycosaminoglycan.

21. The composition of claim 20, wherein said glycosaminoglycan is selected from the group of low molecular weight heparin or heparan derivatives, chemically modified heparin or heparan derivatives and low molecular weight dermatan sulfates and mixtures thereof.

22. The composition of claim 20, wherein said glycosaminoglycan is Sulodexide.

23. A composition for the treatment of fetal growth restriction associated with underlying endothelial dysfunction.

24. The composition of claim 23, wherein said glycosaminoglycan is selected from the group of low molecular weight heparin or heparan derivatives, chemically modified heparin or heparan derivatives and low molecular weight dermatan sulfates and mixtures thereof.

25. The composition of claim 23, wherein said glycosaminoglycan is Sulodexide.
